# EUROPÄISCHE PATENTSCHRIFT

(11) **EP 0 699 730 B1**
(45) Veröffentlichungstag und Bekanntmachung des Hinweises auf die Patenterteilung: **02.11.2000**
(21) Anmeldenummer: 95112915.4
(22) Anmeldetag: 17.08.1995
(51) Int. Cl.: C09K 11/06, H05B 33/14, C07C 229/44, C07C 255/42

(54) **Elektrolumineszierende Anordnungen und deren Verwendung**
Electroluminescent devices and their use
Dispositifs électroluminescents et leur utilisation

(30) Priorität: 30.08.1994 DE 4430691
(43) Veröffentlichungstag der Anmeldung: 06.03.1996
(73) Patentinhaber: BAYER AG, 51368 Leverkusen (DE)
(72) Erfinder: Wehrman, Rolf, Dr., D-47800 Krefeld (DE); Elschner, Andreas, Dr., D-45479 Mülheim (DE); Thurm, Siegfried, Dr., D-40668 Meerbusch (DE); Rosenkranz, Hans Jürgen, Prof. Dr., D-47799 Krefeld (DE); Dujardin, Ralf, Dr., D-47877 Willich (DE)

(56) Entgegenhaltungen:
- EP-A- 0 557 534
- EP-A- 0 595 089

## Beschreibung

Die Erfindung betrifft elektrolumineszierende Anordnungen (Schichten), die spezielle Styrole enthalten und deren Verwendung.

Bei der Entwicklung lichtemittierender Bauteile für Elektronik oder Photonik kommen heute hauptsächlich anorganische Halbleiter wie Galliumarsenid zum Einsatz. Auf Basis derartiger Substanzen können punktförmige Anzeigeelemente hergestellt werden. Großflächige Anordnungen sind nicht möglich.

Neben den Halbleiterleuchtdioden sind elektrolumineszierende Anordnungen auf Basis aufgedampfter niedermolekularer organischer Verbindungen bekannt (z.B. US-A-4 539 507, US-A-5 077 142, EP-A-406 762). Mit diesen Materialien können nur kleindimensionierte LEDs (Light emitting diode) hergestellt werden. Außerdem sind diese elektrolumineszierenden Anordnungen sehr kurzlebig.

Poly-(p-phenylene) und Poly-(p-phenylen)-vinylene (PPV) zeigen Elektrolumineszenz (z.B. Adv. Mater. 4 (1992) Nr. 1; J. Chem. Soc. Chem. Commun. 32 (1992)). Weiterhin ist bekannt, lösliche PPV-Derivate zur Herstellung flexibler Kunststoff-LEDs zu verwenden (z.B. WO 92/16023).

Es wurde nun gefunden, daß bestimmte substituierte Styrole elektrolumineszierende Eigenschaften zeigen, wenn sie in entsprechende Anordnungen eingebracht werden.

Gegenstand der Erfindung sind elektrolumineszierende Anordnungen, dadurch gekennzeichnet, daß als elektrolumineszierende Substanz Styrole der Formel (I) 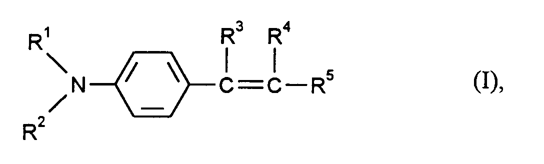 in welcher
- R¹ und R²: unabhängig voneinander C₁-C₂₀-Alkyl, C₆-C₁₄-Aryl, C₇-C₁₅-Alkylaryl und C₇-C₁₅-Arylalkyl,
- R³: Wasserstoff, C₁-C₂₀-Alkyl, C₆-C₁₄-Aryl, C₇-C₁₅-Alkylaryl, C₇-C₁₅-Arylalkyl , Halogen und -CN,
- R⁴ und R⁵: unabhängig voneinander für -CN, COOH gegebenenfalls mit OH-substituiertes C₁-C₂₀-Carboxylat bedeuten,
eingesetzt werden.

In Formel (I) stehen R¹ und R² vorzugsweise für Methyl, Ethyl, Propyl, Butyl, Pentyl, Hexyl, Heptyl, 2-Ethylhexyl, Octyl, Decyl, Dodecyl, Myristyl, Cetyl, Stearyl, Phenyl, Benzyl,

R³ vorzugsweise für Wasserstoff, Methyl, Ethyl, Propyl, Butyl, Pentyl, Hexyl, Heptyl, 2-Ethylhexyl, Octyl, Decyl, Dodecyl, Myristyl, Cetyl, Stearyl, Phenyl, Benzyl, Alkylphenyl, Fluor, Chlor, Brom, Iod, Nitril (CN),
- R⁴ und R⁵: vorzugsweise für Nitril, COOH, COOMe, COOEt, COOProp, COOBu, COOHexyl, COOEthylhexyl, COOOctyl, COODecyl, COODodecyl, COOCetyl, COOHydroxyethyl, COOHydroxypropyl, COOHydroxypropyl, COOHydroxybutyl, COOHydroxyhexyl, COOHydroxyoctyl, COOHydroxydecyl.

Die genannten Alkylderivate der Reste R¹ bis R⁵ können geradkettig oder verzweigt sein.

Beispiele für Styrole der Formel (I) sind:

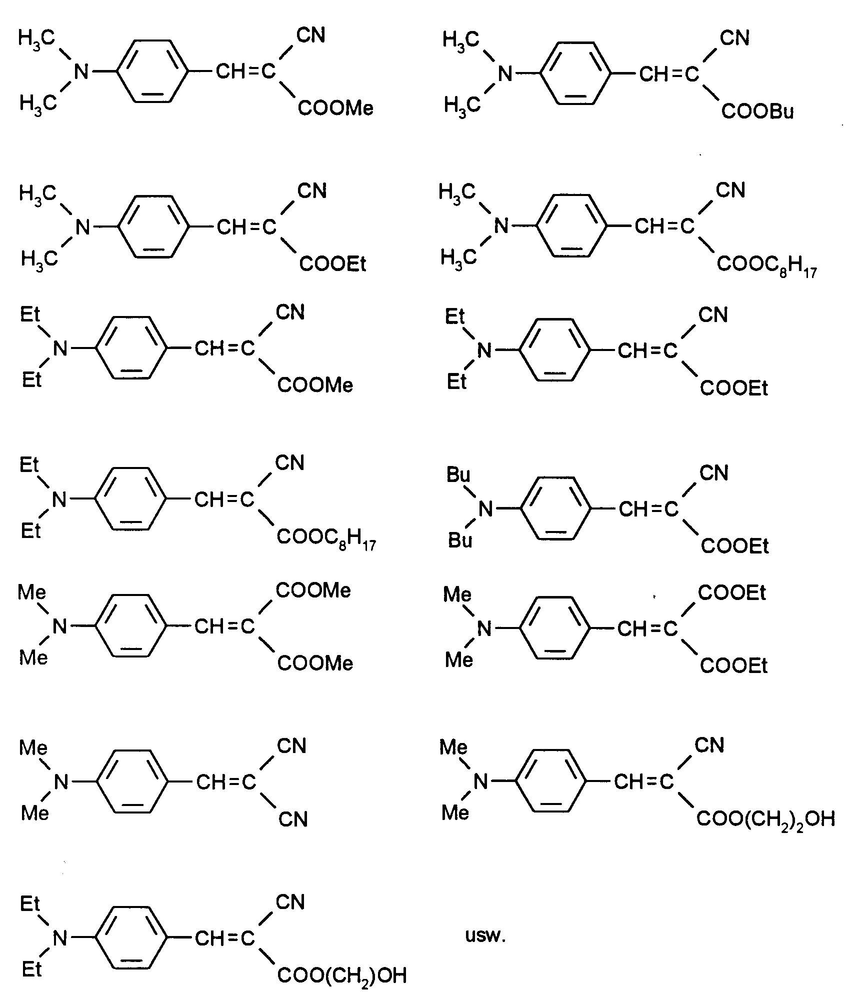

Die erfindungsgemäß einzusetzenden Styrole der Formel (I) sind bekannt und können z.B. nach der Knoevenagel-Reaktion durch Umsetzung von z.B. Cyanessigsäureestern (Nitrilomalonsäureester) der Formel (II) mit N,N-disubstituierten p-Aminobenzaldehyden der Formel (III) erhalten werden, z.B.

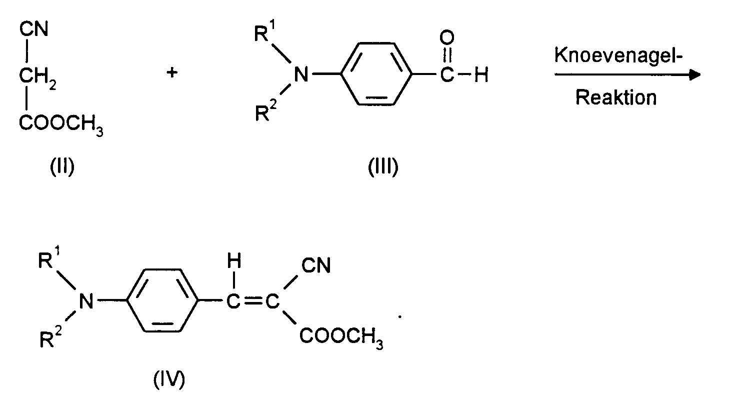

Die Knoevenagel-Reaktion, die Umsetzung von Aldehyden und Ketonen mit CH-Aciden Verbindungen ist z.B. ausführlich in "Organikum", VEB Deutscher Verlag der Wissenschaften, Berlin 1977, beschrieben.

Bei den erfindungsgemäßen Systemen befindet sich die elektrolumineszierende (EL) Schicht zwischen zwei Elektroden. Mindestens eine der beiden Elektroden ist im sichtbaren Spektralbereich transparent.

Eine transparente Elektrode wird auf einen transparenten Träger, z.B. Glas, Kunststoffmaterial (z.B. PET-Folie), aufgebracht. Als transparente Elektrode (R) sind geeignet
a) Metalloxide, z.B. Indium-Zinn-Oxid (ITO), Zinnoxid (NESA), etc.,
b) semi-transparente Metallfilme, z.B. Au, Pt, Ag, Cu etc.,
c) leitfähige Polymerfilme wie Polyaniline, Polythiophene, etc.

Die Metalloxid- und die semitransparenten Metallfilmelektroden werden durch Techniken wie Aufdampfen, Aufsputtern, Platinierung, etc., in dünner Schicht aufgebracht. Die leitfähigen Polymerfilme werden durch Techniken wie Spincoaten, Casing, Rakeln etc. aus der Lösung aufgebracht.

Die Dicke der transparenten Elektrode beträgt 50 Å bis etwa mehrere µm, vorzugsweise 100 Å bis 5 000 Å.

Die Styrole der Formel (I) als elektrolumineszierende Substanz werden auf die transparente Elektrode als dünner Film aufgebracht. Die Dicke des Films beträgt 30 Å bis 10 µm, vorzugsweise 100 Å bis 1 µm.

Nach dem Trocknen der EL-Schicht wird diese mit einer Gegenelektrode ausgerüstet. Diese besteht aus einer leitfähigen Substanz, die transparent sein kann. Vorzugsweise eignen sich Metalle wie Al, Au, Ag, etc. oder Legierungen und Oxide dieser, die durch Techniken wie Aufdampfen, Aufsputtern, Platinierung aufzubringen sind.

Die erfindungsgemäße Anordnung wird durch zwei elektrische Zuführungen (z.B. Metalldrähte) mit den beiden Elektroden in Kontakt gebracht.

Die Anordnungen emittieren beim Anlegen einer Gleichspannung im Bereich von 1 bis 100 Volt Licht der Wellenlänge von 400 bis 700 nm. Sie zeigen im Bereich von 400 bis 700 nm Photolumineszenz.

Die elektrolumineszierende Schicht kann eine oder mehrere Styrole der Formel (I) enthalten. Sie enthält gegebenenfalls weiterhin übliche Zusätze wie inerte Binder, ladungsträgertransportierende Substanzen, Mischungen aus inerten Bindern und ladungsträgertransportierenden Substanzen. Ladungsträgertransportierende Substanzen erhöhen die Elektrolumineszenzintensität und reduzieren die Einsatzspannungen.

Als inerte Binder werden vorzugsweise lösliche transparente Polymere wie z.B. Polycarbonate, Polystyrol und Copolymere des Polystyrols wie SAN, Polysulfone, Polyacrylate, Polyvinylcarbazol, Vinylacetat- und Vinylalkoholpolymere und -copolymere usw. eingesetzt.

Zwischen den elektrolumineszierenden Systemen und den Elektroden können zusätzlich eine oder mehrere Zwischenschichten angeordnet sein. Diese Zwischenschichten - ladungsträgertransportierende Substanzen - sind bekannt (z.B. aus Appl. Phys. Lett. 57 (1990) 531) und werden dort als HTL (hole transport layer) und als ETL (electron transport layer) bezeichnet. Diese Zwischenschichten erhöhen die Elektrolumineszenzintensität.

Die erfindungsgemäßen Anordnungen können zur Herstellung von Leuchtanzeigen (displays), auch in großen Formaten, verwendet werden.

### Beispiele

### a) Herstellung eines OH-substituierten Cyanessigesters durch Umsetzung eines Cyanessigsäureesters mit Ethylenglykol

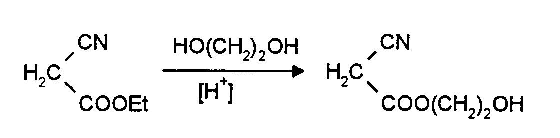

4 mol Cyanessigsäureethylester werden mit 4 mol Ethylenglykol und 3 g p-Toluolsulfonsäure auf Rückfluß erhitzt. Es spaltet sich Ethanol ab, das über eine Kolonne abgetrennt wird. Der Rückstand wird im Hochvakuum destilliert. Das Produkt wird über sein NMR-Spektrum charakterisiert.

### b) Herstellung eines langkettigen Cyanessigsäureesters durch Umsetzung von Cyanessigsäuremethylester mit 2-Ethylhexanol

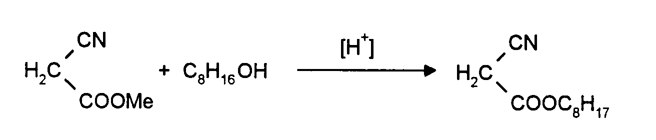

4 mol Cyanessigsäuremethylester werden mit 4,4 mol 2-Ethylhexanol und 3 g p-Toluolsulfonsäure so lange unter Rückfluß erhitzt, bis die theoretische Menge an Methanol abgespalten und abdestilliert worden ist. Der Rückstand wird im Wasserstrahlvakuum fraktioniert.

Auf analoge Weise werden die Butyl-, Hexyl- und Benzylester gewonnen.

### c) Umsetzung von Cyanessigsäureestern mit N,N-Dialkylaminobenzaldehyden nach Koevenagel zu 2-4-(N,N-Dimethylaminophenyl)cyanacrylsäurehydroxyethylester

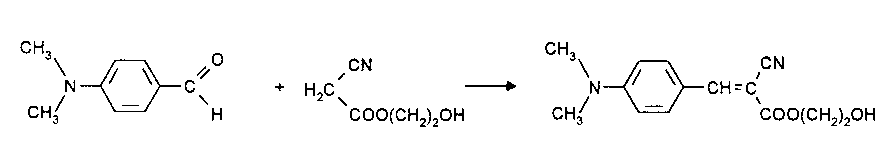

0,75 mol Dimethylaminobenzaldehyd und 0,75 mol des Cyanessigsäureesters aus a) werden unter Zusatz von 1,5 g Piperidin und 1,2 g Eisessig in 200 ml Toluol so lange unter Rückfluß am Wasserabscheider erhitzt, bis die theoretische Menge an Wasser abgetrennt ist.

Das nach dem Abkühlen ausgefallene Produkt wird mit Wasser und anschließend mit Petrolether gewaschen und aus Toluol umkristallisiert. ¹H-NMR (ppm): 8,12 CH=; 4,22 und 3,69 OCH₂; CH₃N.

Auf analoge Weise werden die Cyanessigester aus Beispiel b) umgesetzt.

### d) Elektrolumineszierende Anordnung I

ITO beschichtetes Glas (hergestellt von der Firma Balzers) wird in 20 x 30 mm große Stücke geschnitten und in folgenden Schritten gereinigt:
1. 15 min in dest. Wasser/Falterol-Spülmittel (basisch) im Ultraschallbad,
2. 2 x 15 min in jeweils frischem dest. Wasser im Ultraschallbad spülen,
3. 15 min in Ethanol im Ultraschallbad,
4. 2 x 15 min in jeweils frischem Aceton im Ultraschallbad,
5. Trocknen auf fusselfreien Linsentüchern.

0,45 Gew.-Teile Poly(9-vinylcarbazol) Aldrich Chemical Co. Ltd. #18, 260-5) und 1,05 Gew.-Teile des elektrolumineszierenden Styrols der Formel (I) mit R¹ = CH₃, R² = CH₃, R³ = H, R⁴ = CN, R⁵ = COOEthylhexyl werden in Dichlorethan gelöst. Die Lösung wird gefiltert (0,2 µm-Filter). Die gefilterte Lösung wird mit einer Lackschleuder bei 1000 U/min auf dem ITO-Glas verteilt. Die Dicke des trockenen Films beträgt 230 nm und der Ra-Wert der Oberfläche beträgt 10 nm (Stylusprofilometer Alpha-Step 200 der Firma Tencor Inst.).

Der so hergestellte Film wird anschließend mit Al-Elektroden bedampft. Dazu werden mit Hilfe einer Lochmaske isolierte Al-Punkte mit einem Durchmesser von 3 mm auf den Film gedampft. Während des Aufdampfens herrscht in einer Aufdampfapparatur ein Druck p < 10-5 mbar.

Die ITO-Schicht und die Al-Elektrode wird über elektrische Zuführungen mit einer Spannungsquelle verbunden. Beim Erhöhen der Spannung fließt ein elektrischer Strom durch die Anordnung und die beschriebene Schicht elektroluminesziert. Die Elektrolumineszenz tritt dabei unabhängig von der Polung der angelegten Spannung auf.

Die Leuchtdichte der Elektrolumineszenz beträgt bei 25 Volt 100 cd/m² (Minolta, LS 100).

### e) Elektrolumineszierende Anordnung II

Gemäß Herstellung der elektrolumineszierenden Anordnung I wurde die elektrolumineszierende Schicht aus einer Dichlorethanlösung mit 0,7 Gew.-Teilen an Polystyrol und 0,3 Gew.-Teilen eines Styrols der Formel (I) mit R¹ = CH₃, R² = CH₃, R³ = H, R⁴ = CN und R⁵ = CN, hergestellt.

Die Leuchtdichte der Elektrolumineszenz beträgt bei 40 Volt 5 cd/m².

### f) Weitere elektrolumineszierende Anordungen

Gemäß der Herstellung der elektrolumineszierenden Anordungen I und II wurden elektrolumineszierende Schichten aus 0,3 Gew.-Teilen an polymeren Bindern und 0,7 Gew.-Teilen eines Styrols der Formel I mit R₁ = CH₃, R₂ = CH₃, R₃ = H, R₄ = CN und R₅ = COOCH₃ hergestellt. Die Schichtdicken betrugen jeweils etwa 150 nm. Als polymere Binder wurden Polyvinylcarbazol (PVK), Styrol-Acrylnitril (SAN), Polymethylmethacrylat (PMMA), Polystyrol (PS) und Polycarbonat (PC) verwendet.

Alle Anordnungen zeigen Elektrolumineszenz ab einer angelegten Spannung von ca. 15 Volt.

## Patentansprüche

1. Elektrolumineszierende Anordnungen, dadurch gekennzeichnet, daß als elektrolumineszierende Substanz Styrole der Formel (I) 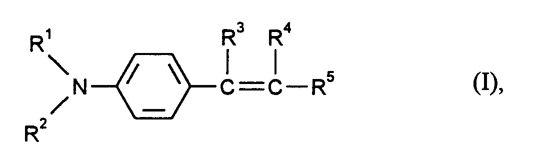 in welcher
R¹ und R² unabhängig voneinander C₁-C₂₀-Alkyl, C₆-C₁₄-Aryl, C₇-C₁₅-Alkylaryl und C₇-C₁₅-Arylalkyl,
R³ Wasserstoff, C₁-C₂₀-Alkyl, C₆-C₁₄-Aryl, C₇-C₁₅-Alkylaryl, C₇-C₁₅-Arylalkyl , Halogen und -CN,
R⁴ und R⁵ unabhängig voneinander für -CN, -COOH gegebenenfalls mit OH-substituiertem C₁-C₂₀-Carboxylat bedeuten,
eingesetzt werden.

2. Verwendung von elektrolumineszierenden Anordnungen nach Anspruch 1 zur Herstellung von Leuchtanzeigen.

## Claims

1. Electroluminescent arrangements, characterised in that styrenes of the formula (I) 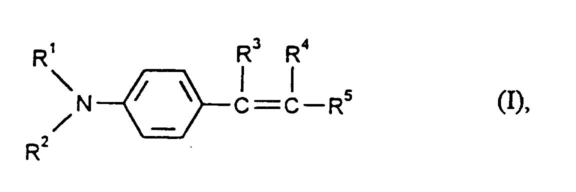 in which
R¹ and R² mutually independently represent C₁-C₂₀ alkyl, C₆-C₁₄ aryl, C₇-C₁₅ alkylaryl and C₇-C₁₅ arylalkyl,
R³ represents hydrogen, C₁-C₂₀ alkyl, C₆-C₁₄ aryl, C₇-C₁₅ alkylaryl, C₇-C₁₅ arylalkyl, halogen and -CN,
R⁴ and R⁵ mutually independently represent -CN, -COOH, optionally OH-substituted C₁-C₂₀ carboxylate,
are used as the electroluminescent substance.

2. Use of electroluminescent arrangements according to claim 1 for the production of luminous displays.

## Revendications

1. Dispositifs électroluminescents caractérisés en ce qu'on met en oeuvre, à titre de substance électroluminescente, des styrènes répondant à la formule (I) 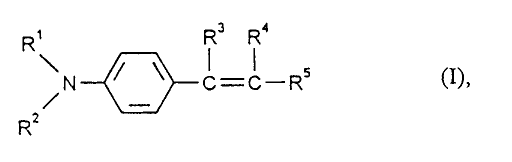 dans laquelle
R¹ et R² représentent, indépendamment l'un de l'autre, un groupe alkyle en C₁-C₂₀, un groupe aryle en C₆-C₁₄, un groupe alkylaryle en C₇-C₁₅ et un groupe arylalkyle en C₇-C₁₅,
R³ représente un atome d'hydrogène, un groupe alkyle en C₁-C₂₀, un groupe aryle en C₆-C₁₄, un groupe alkylaryle en C₇-C₁₅, un groupe arylalkyle en C₇-C₁₅, un atome d'halogène et un groupe -CN,
R⁴ et R⁵ représentent, indépendamment l'un de l'autre, un groupe -CN, un groupe -COOH, carboxylate de radical hydrocarboné en C₁-C₂₀ éventuellement substitué par un groupe OH.

2. Utilisation de dispositifs électroluminescents selon la revendication 1 pour la fabrication de systèmes électriques d'affichage.
